(19) Europäisches Patentamt / European Patent Office / Office européen des brevets

(11) **EP 2 345 738 A1**

(12) **EUROPEAN PATENT APPLICATION**

(43) Date of publication:
**20.07.2011 Bulletin 2011/29**

(51) Int Cl.:
**C12Q 1/68** (2006.01)

(21) Application number: **10012272.0**

(22) Date of filing: **05.08.2003**

(84) Designated Contracting States:
**AT BE BG CH CY CZ DE DK EE ES FI FR GB GR HU IE IT LI LU MC NL PT RO SE SI SK TR**

(30) Priority: **05.08.2002 US 400685 P**

(62) Document number(s) of the earlier application(s) in accordance with Art. 76 EPC:
**03767188.0 / 1 540 009**

(71) Applicant: **Quanta Biosciences, Inc.
Gaithersburg, MD 20877 (US)**

(72) Inventors:
• **Rashtchian, Ayoub
Gaithersburg, MD 20882 (US)**
• **Schuster, David M.
Poolesville, MD 20837 (US)**

(74) Representative: **Bohmann, Armin K.
bohmann
Anwaltssozietät
Nymphenburger Strasse 1
80335 München (DE)**

Remarks:
This application was filed on 30-09-2010 as a divisional application to the application mentioned under INID code 62.

(54) **Improved compositions for in vitro amplification of nucleic acids**

(57) Method and compositions for improving DNA polymerase and reverse transcriptase reactions are provided. Addition of anti-foam reagents to the reactions improves fluid handling, especially of small volumes and allows enhanced accuracy of optical detection, without substantially inhibiting enzymatic activity.

EP 2 345 738 A1

# Description

This application claims priority to U.S. provisional application serial number 60/400,685, filed August 05, 2002, the entirety of which is hereby incorporated by reference.

## FIELD OF THE INVENTION

The invention provides improved methods for detecting and amplifying nucleic acid molecules. More specifically, the invention provides methods for nucleic acid amplification that employ anti-foam reagents to improve fluidic handling and provide enhanced accuracy of real-time optical monitoring of amplification reaction mixtures.

## BACKGROUND

The polymerase chain reaction (PCR) is a fundamental technique in molecular biology for the amplification of nucleic acid sequence in biological samples (Mullis, K. et al., Cold Spring Harbor Symp. Quant. Biol. 51:263-273 (1986); Erlich H. et al., EP 50,424; EP 84,796, EP 258,017, EP 237,362; Mullis, K., EP 201,184; Mullis K. et al., U.S. Pat. No. 4,683,202; Erlich, H., U.S. Pat. No. 4,582,788; and Saiki, R. et al., U.S. Pat. No. 4,683,194) , which references are incorporated herein by reference). PCR achieves the amplification of a specific nucleic acid sequence using two oligonucleotide primers complementary to regions of the sequence to be amplified. Extension products incorporating the primers then become templates for subsequent replication steps.

PCR provides a method for selectively increasing the concentration of a nucleic acid molecule having a particular sequence even when that molecule has not been previously purified and is present only in a single copy in a particular sample. The method can be used to amplify either single or double stranded DNA.

Typically, nucleic acid analysis by PCR requires sample preparation, template amplification, and product analysis by agarose gel electophoresis or hybridization assay. A typical PCR reaction by itself only yields qualitative data, since, after a phase of exponential or progressive amplification, the amount of amplified nucleic acid reaches a plateau, such that the amount of generated reaction product is not proportional to the initial concentration of the template DNA. Consequently, many different PCR based protocols have been developed to obtain reliable and reproducible quantitative data. In general, quantification of analyte at PCR plateau has required either the generation of calibration curves, reviewed by Siebert, in: Molecular Diagnosis of infectious diseases (ed. Reiscbl, Humana Press, Totowa, N.J., p. 55-79 (1998), or competitive PCR using internal standards. Both these approaches are time consuming and require multiple amplification reactions to quantify a specific nucleic acid sequence present in a single sample.

Alternately, Wiesner et al.(Nucl. Acids Res. 20, 5863-5864 (1992)), used data from multiple cycles of a PCR reaction, where after each cycle the product concentration was assayed by radioactive incorporation and subsequent scintillation counting. For each curve, the initial template concentration (No) and amplification efficiency (eff) were determined by linear regression of data points on a product concentration (Nn) versus cycle number graph as defined by the following formula:

$$\mathrm{Log\ Nn} = (\log \mathrm{eff})\mathrm{n} + \log \mathrm{N_o}.$$

A major improvement in the generation of quantitative data derives from the possibility of measuring the kinetics of a PCR reaction by on-line detection. This has become possible recently by detecting the amplicon through fluorescence monitoring and measurement of PCR product by fluorescent dual-labeled hybridization probe technologies, such as the "TaqMan" 5' fluorogenic nuclease assay described by Holland et al. (Proc. Natl. Acad. Sci. U.S.A. 88, 7276 (1991)), Gibson et al. (Genome Res. 6, 99 (1996)), and Heid et al. (Genome Res. 6, 986 (1996)); or "Molecular Beacons" (Tyagi, S. and Kramer, F.R. Nature Biotechnology 14, 303 (1996)). Nazarenko et al. (Nucleic. Acids Res. 25, 2516 (1997)) have described use of dual-labeled hairpin primers, as well as recent modifications utilizing primers labeled with only a single fluorophore (Nazerenko et al., Nucleic. Acids Res. (2002)). One of the more widely used methods is the addition of double-strand DNA-specific fluorescent dyes to the reaction such as: ethidium bromide (Higuchi et al., Biotechnology (1992) and Higuchi et al., Biotechnology 11, 102610, 413 (1993)), YO-PRO-1 (Ishiguro et al., Anal. Biochem. 229, 207 (1995)), or SYBR Green I (Wittwer et al., Biotechniques 22,130 (1997)). These improvements in the PCR method have enabled simultaneous amplification and homogeneous detection of the amplified nucleic acid without purification of PCR product or separation by gel electrophoresis. This combined approach decreases sample handling, saves time, and greatly reduces the risk of product contamination for subsequent reactions, as there is no need to remove the samples from their closed containers for further analysis. The concept of combining amplification with product analysis has become known as "real time" PCR.

The general principals for template quantification by real-time PCR were first disclosed by Higuchi R, G Dollinger, P S Walsh and R. Griffith, "Simultaneous amplification and detection of specific DNA sequences", Bio/Technology 10:413-417, 1992; Higuchi R, C Fockler G Dollinger and R Watson, Kinetic PCR analysis: real time monitoring of DNA amplification reactions, Bio/Technology 11:1026-1030. This simpler approach for quantitative PCR utilizes a double-strand specific fluorescent dye, ethidium bromide, added to amplification reaction. The fluorescent signal generated at each cycle of PCR is proportional to the amount of PCR product. A plot of fluorescence versus cycle number is used to describe the kinetics of amplification and a fluorescence threshold level was used to define a fractional cycle number related to initial template concentration. Specifically, the log of the initial template concentration is inversely proportional to the fractional cycle number (threshold cycle, or Ct), defined as the intersection of the fluorescence versus cycle number curve with the fluorescence threshold. Higher amounts of starting template results in PCR detection at a lower Ct value, whereas lower amounts require a greater number of PCR cycles to achieve an equivalent fluorescent threshold (Ct) and are detected at higher Ct values. Typically, the setting of this fluorescence threshold is defined as a level that represents a statistically significant increase over background fluorescent noise.

A major problem in automating PCR data analysis is identification of baseline fluorescence. Background fluorescence varies from reaction to reaction. Moreover, baseline drift, wherein fluorescence increases or decreases without relation to amplification of nucleic acids in the sample, is a common occurrence. These problems are often exacerbated by bubbles in the reaction that interfere with optical measurements. The bubbles likely are caused by the presence of non-ionic polymeric detergents, which are a necessary component of the amplification reaction. Gelfand et al. have disclosed use of non-ionic detergent, typically selected from the group consisting of octoxynol, polyoxyethylated sorbitan monolaurate and ethoxylated nonyl phenol, to stabilize thermostable enzymes used for PCR have been disclosed by Gelfand et al. (US Pat. No. 6,127,155). Karsai et al., BioTechniques 32, 790 (2002) reported that 1.5% Triton X-100 was a critical component in SYBR Green I real-time PCR. Therefore, it would be advantageous to have an amplification reaction that is stabilized, but free of optical interference from bubbles.

In addition to PCR amplification, there are other enzymatic reactions involving nucleic acids that use detergents as reaction enhancers or as stabilizing agents. Examples of these include T7 RNA polymerase (Ambion, Catalog number 2716) using Tween-20; Superscript II reverse transcriptase (Invitrogen, Catalog number 18064-022) using NP-40; and AMV reverse transcriptase (FinnZyme, Catalog Number F-570S or Seikagaku America, Catalog number 12048-2) using Triton X-100.

A major problem in understanding of gene expression patterns for gene discovery and identification of metabolic pathways is the limitations of current methods for accurate quantification. Use of real time PCR methods provides a significant improvement towards this goal, however, limitations in accurate liquid handling and delivery for assembly of real time PCR reactions still present a significant problem. This problem is exacerbated, moreover, when dealing with small volumes required for high through put analyses. These limitations are also applicable to arrange of molecular analyses involving other enzymatic reactions such as cDNA synthesis by reverse transcriptases or RNA synthesis and amplification by in vitro transcription. This invention provides methods for solving this problem by use of anti-foam agents that reduce or eliminate foaming and that improve accurate delivery of small volumes of reagents in high through put experimentation.

## SUMMARY OF THE INVENTION

The instant invention relates to the use of anti-foam agents in enzymatic reactions, in particular in *in vitro* nucleic acid amplification reactions and, more particularly, in homogenous phase or real time reactions that exploit optical detection of a fluorescent signal to quantify and detect amplification product. The present invention provides methods and compositions for improving the accuracy of optical detection in real time PCR by eliminating interfering factors such as bubbles commonly encountered in real time PCR. The invention also provides methods of detecting the amplification of a target nucleic acid that reduces variation in background fluorescence. The invention also provides kits for enzymatic reactions, including kits for amplification of nucleic acid sequence, that incorporate the anti-foam moiety described herein.

The anti-foam compounds described herein are also useful for decreasing variation in the fluorescence background in enzyme assays, by reducing optical interference from bubbles that would otherwise change the fluorescent signal in a manner that is not dependent on the concentration of the desired nucleic acid product.

In other embodiments of the invention methods and compositions are described for improvement of accuracy and ease of liquid handling for assembly of enzymatic reactions. The present invention describes use of certain anti-foam agents in specific concentration ranges that effectively reduce foaming of buffers and reagents used in PCR without interference in enzymatic activity required for effective amplification. In further embodiments, the anti-foam compounds are useful for the amplification of a nucleic acid template by the polymerase chain reaction (PCR), real-time/kinetic PCR, reverse transcription PCR (RT PCR), linked linear amplification (U.S. Pat. No. 6,027,923 to Wallace (2000), incorporated by reference), the ligase chain reaction (LCR, PROCEEDINGS OF THE NATIONAL ACADEMY OF SCIENCE, USA 88, 189 (1991), incorporated by reference), nucleic acid sequence-based amplification (NASBA, NATURE 350, 91 (1991), incorporated by reference), Q beta replicase-based amplification, cycling probe reaction CPR), solid phase amplification (SPA), self-sustained sequence replication (3SR, PROCEEDINGS OF THE NATIONAL ACADEMY OF SCIENCE, USA 87, 1874 (1990), incorporated by reference), terminal transferase-based elongation, or telomerase assays. In other embodiments, the compounds of the present invention are useful in enzymatic reactions involving RNA polymerases and cDNA synthesis reactions using reverse transcriptases. It is important to note that RNA polymerases and reverse transcriptases have been used extensively in methods for amplification of RNA or DNA. Examples of these amplification methods are NASBA and 3SR.

Specifically, the invention provides methods for detecting a target nucleic acid in a sample, comprising of amplifying the target nucleic acid using a polymerase chain reaction, where the polymerase chain reaction is carried out in the presence of an effective amount of at least one anti-foam reagent that does not substantially inhibit the action of the polymerase. Mixtures of two or more anti-foam agents also may be used. The polymerase chain reaction may be a quantitative polymerase chain reaction. The polymerase chain reaction also may be a reverse transcriptase polymerase chain reaction. The methods exclude the use of antifoam reagents in strand displacement amplification (SDA). The compositions of the invention also exclude compositions containing mixtures of BsoB1 and Bst polymerases.

The methods described above may be carried out in a sample chamber of a device comprising a plurality of said sample chambers. Each of a plurality of such sample chambers of such a device may contain reagents suitable for detecting a target nucleic acid, and/or for detecting different target nucleic acids.

In these methods the product of the polymerase chain reaction may be detected by optical detection, for example by using a probe labeled with a detectable label, such as a fluorescent dye. The dye may be a fluorescent nucleic acid-binding dye.

The invention also provides compositions for amplifying a target nucleic acid, comprising at least one primer molecule that hybridizes to the target nucleic acid, nucleotide triphosphates, a thermostable DNA polymerase, a detergent, and an effective amount of at least one anti-foam reagent that does not substantially inhibit the action of said thermostable DNA polymerase. Compositions comprising at least two anti-foam reagents also may be used.

In all these methods and compositions, the anti-foam agent (or mixture of anti-foam reagents) may be selected from the group consisting of 1520-US, AF, FG-10, O-30, SE-15, Antifoam B, and the reagents described below in the section headed "Anti-foam."

## BRIEF DESCRIPTION OF THE DRAWINGS

Table 1 shows the effect of Dow 1520-US anti-foam on Taqman real-time quantitative PCR. Summary of Ct values for Taqman PCRs containing varying amounts of anti-foam and DNA target as indicated in the table and corresponding linear regression analysis for the Ct versus log DNA input standard curve.

Table 2 shows the effect of anti-foam compounds on TaqMan real-time quantitative PCR. Summary of Ct values for Taqman PCRs containing various anti-foam compounds, or control reactions that omit anti-foam (CNTRL), and DNA target as indicated in the table and corresponding linear regression analysis for the Ct versus log DNA input standard curve.

Table 3 shows the effect of anti-foam compounds on SYBR Green I real-time quantitative PCR. Summary of Ct values for real-time PCRs containing various compounds, or control reactions that omit (CNTRL), and DNA target as indicated in the table and corresponding linear regression analysis for the Ct versus log DNA input standard curve.

Table 4 shows the effect of antifoam agents on in vitro transcription using T7 RNA polymerase.

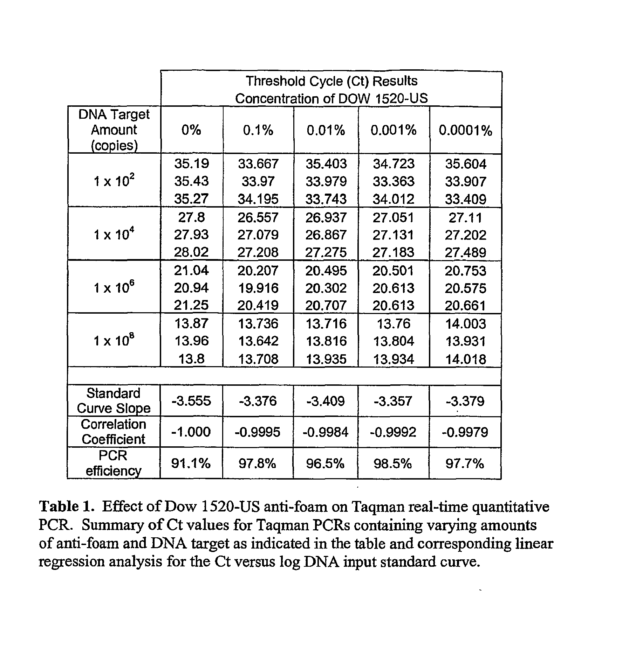

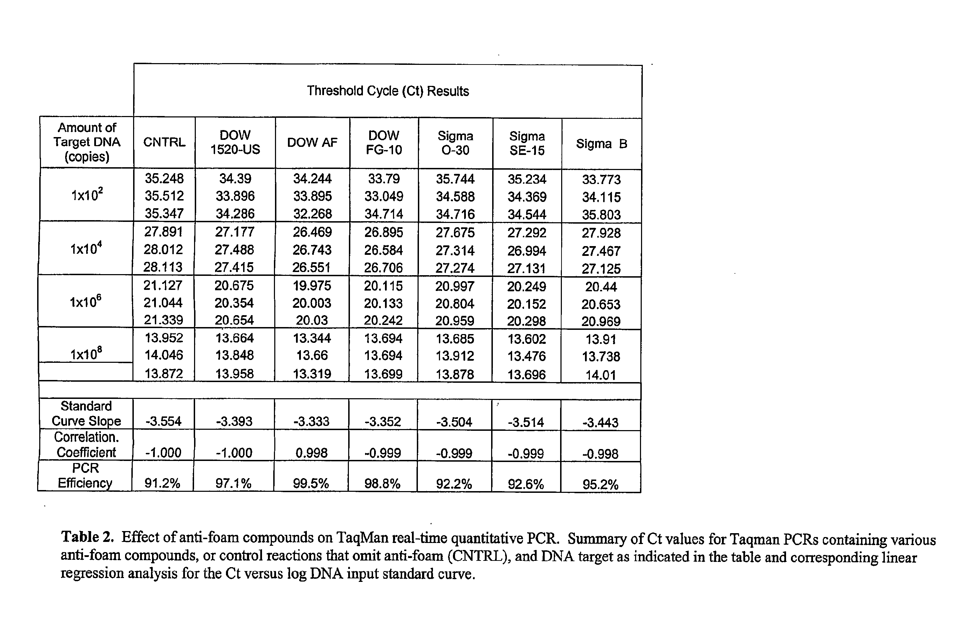

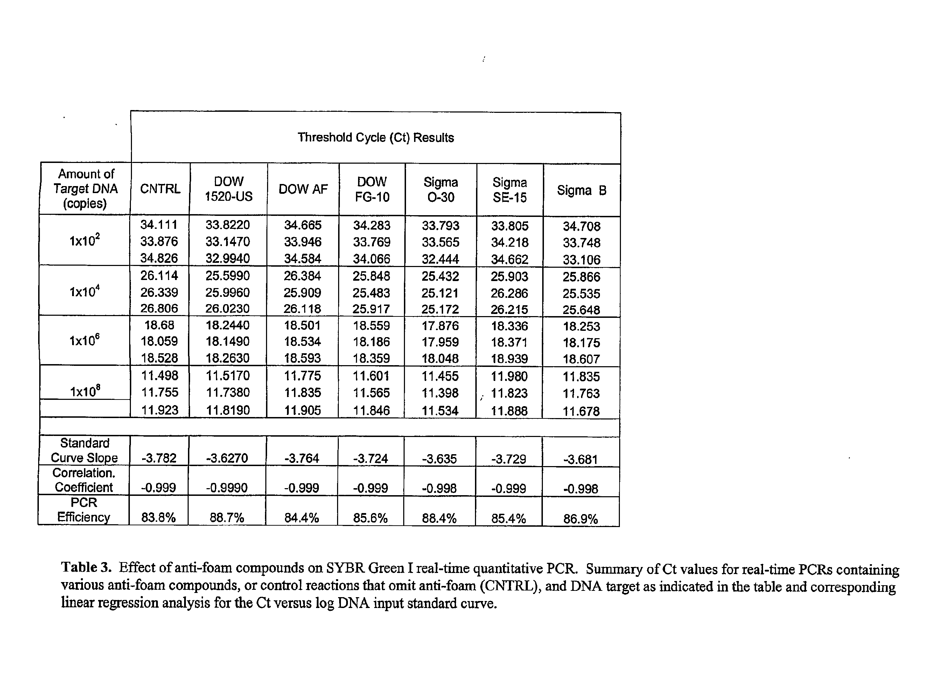

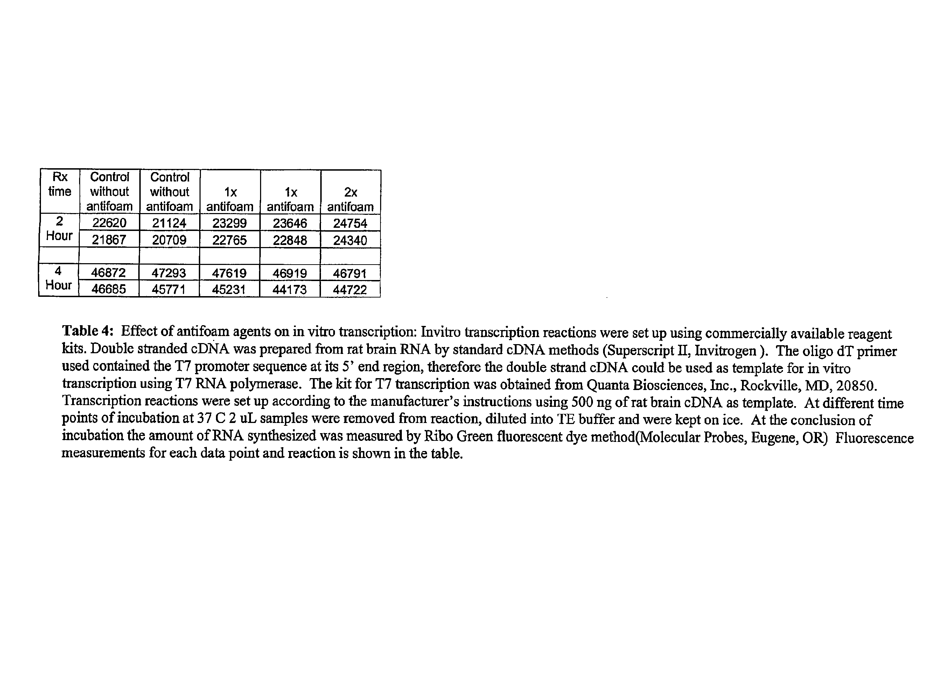

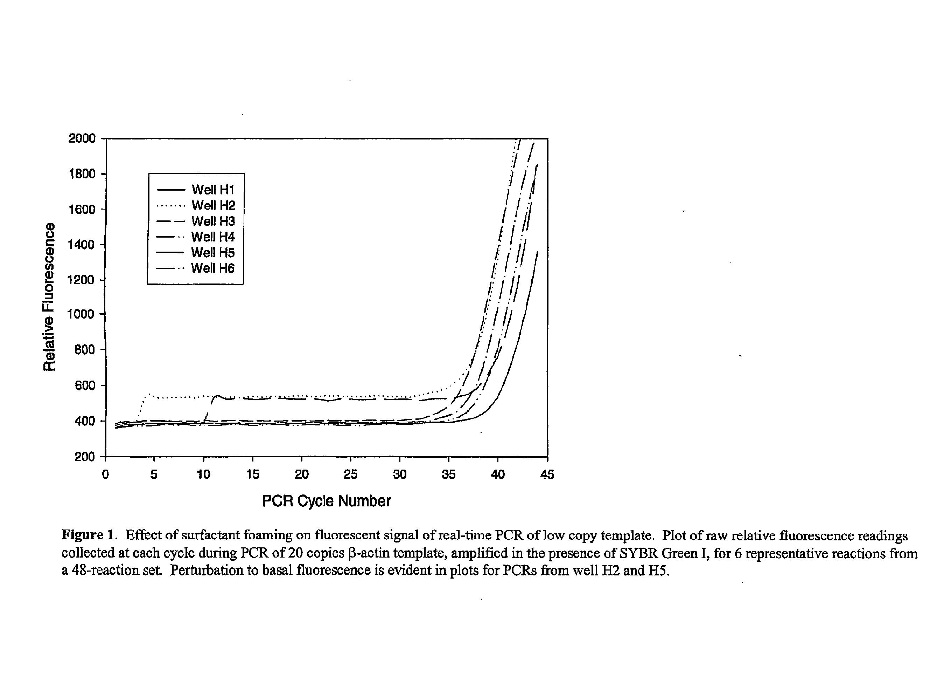

Figure 1 shows the effect of surfactant foaming on fluorescent signal of real-time PCR of low copy template. Plot of raw relative fluorescence readings collected at each cycle during PCR of 20 copies β-actin template, amplified in the presence of SYBR Green I, for 6 representative reactions from a 48-reaction set. Perturbation to basal fluorescence is evident in plots for PCRs from well H2 and H5.

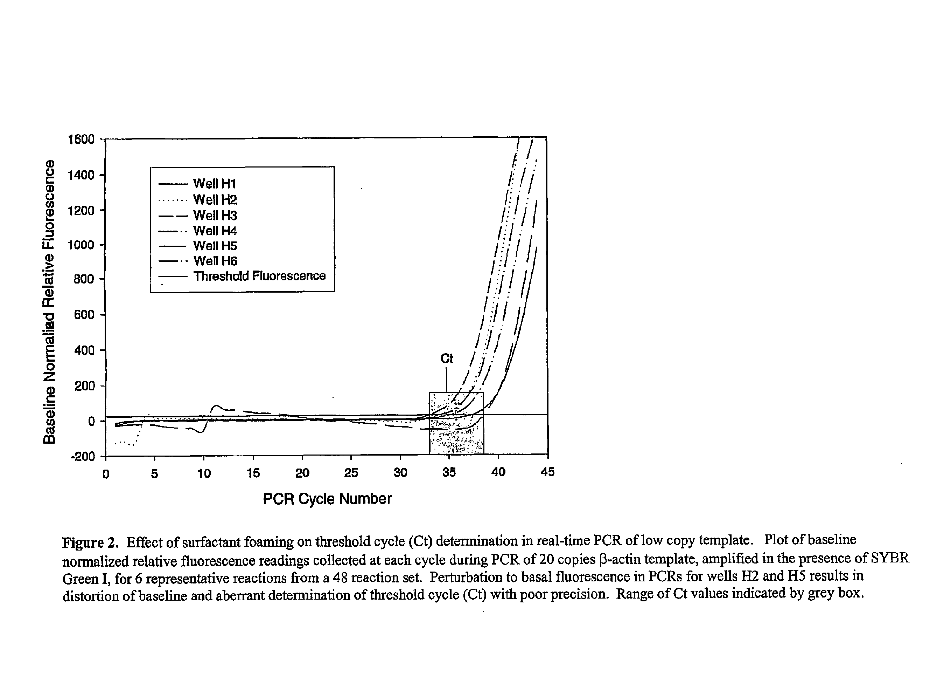

Figure 2 shows the effect of surfactant foaming on threshold cycle (Ct) determination in real-time PCR of low copy template. Plot of baseline normalized relative fluorescence readings collected at each cycle during PCR of 20 copies β-actin template, amplified in the presence of SYBR Green I, for 6 representative reactions from a 48 reaction set. Perturbation to basal fluorescence in PCRs for wells H2 and H5 results in distortion of baseline and aberrant determination of threshold cycle (Ct) with poor precision. Range of Ct values indicated by grey box.

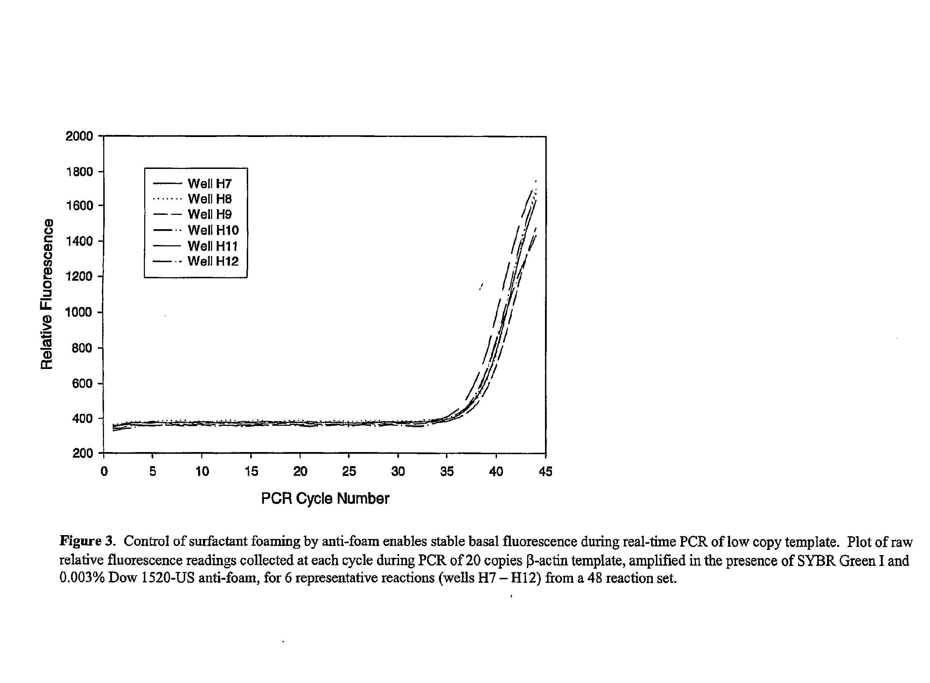

Figure 3 shows that control of surfactant foaming by anti-foam enables stable basal fluorescence during real-time PCR of low copy template. Plot of raw relative fluorescence readings collected at each cycle during PCR of 20 copies β-actin template, amplified in the presence of SYBR Green I and 0.003% Dow 1520-US anti-foam, for 6 representative reactions (wells H7 - H12) from a 48 reaction set.

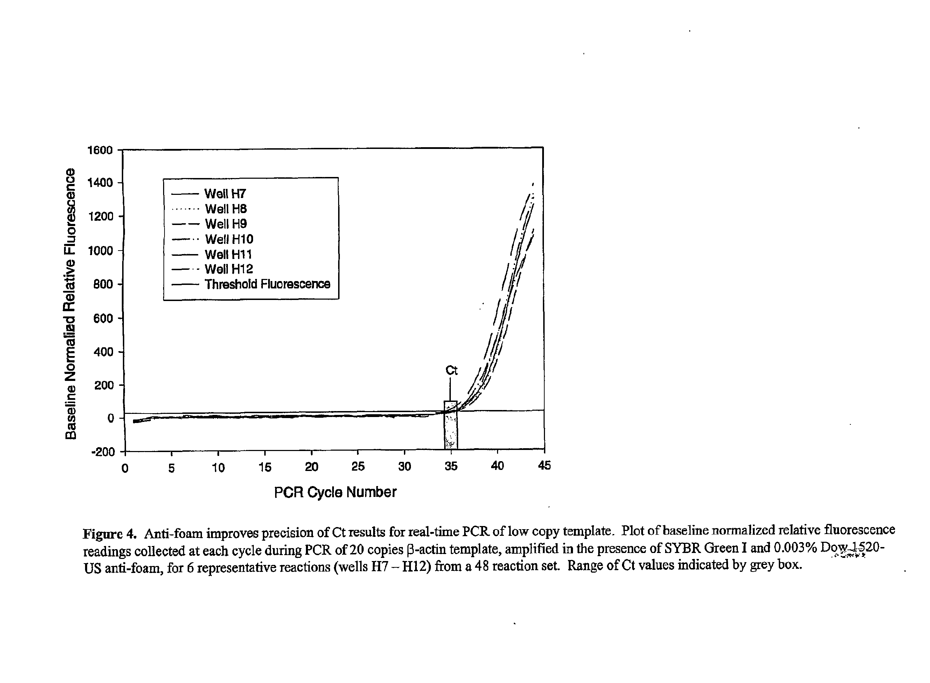

Figure 4 shows how anti-foam improves precision of Ct results for real-time PCR of low copy template. Plot of baseline normalized relative fluorescence readings collected at each cycle during PCR of 20 copies β-actin template, amplified in the presence of SYBR Green I and 0.003% Dow 1520-US anti-foam, for 6 representative reactions (wells H7 - H12) from a 48 reaction set. Range of Ct values indicated by grey box.

## DETAILED DESCRIPTION OF INVENTION:

Methods and compositions are provided for improving the accuracy of optical detection in real time PCR by eliminating interfering factors such as bubbles that are commonly encountered in real time PCR. A variety of anti-foaming agents and a number of combinations of compounds are described that are effective for improving PCR performance. A range of concentrations that effectively reduce or eliminate the liquid handling issues related to detergent containing reaction mixtures also is described. Surprisingly, it has been found that antifoaming agents can be used in a wide range of concentrations without substantially affecting the enzymatic activity of DNA polymerases used in amplification of nucleic acids.

This invention describes addition of anti-foaming agents to amplification reaction compositions commonly used and described in literature. A variety of PCR buffers and mixtures have been used for specific applications of PCR and use of anti-foams is compatible with these formulations. For example: specific buffer compositions have been described and routinely used for amplification of long templates; others are suitable for real time PCR, SYBR green detection real time PCR, realtime PCR using fluorogenic probes, and One step RT PCR. Activity and stability of Taq DNA polymerase during PCR is dependent on the presence of optimal amounts of non-ionic detergents.

Surprisingly, it has been found that the presence of anti-foam agents not only improves handling and accuracy of pipetting, but also improves performance of amplification reagents in a variety of formulated buffers. For example a "mastermix" prepared from a commercially available product, iQ PCR SYBR Green SuperMix (Bio-Rad Laboratories), may successfully be used with anti-foam agent as described, for example, in example IV. Reaction mixtures containing anti foam also can be formulated using separate component solutions as compared to a ready-to-use mastermix. For example, in example V, each 50-$\mu$l PCR contains 1.25 units of iTaq DNA polymerase (Bio-Rad Laboratories), 1X PCR buffer (20 mM Tris-HCl, pH 8.4, 50 mM KCl), 3 mM magnesium chloride, 0.2 mM each dNTP, 200 nM each primer, 100 nM FAM and TAMRA-labeled probe. PCRs is conducted under identical conditions except for the inclusion of varying amounts (0.1%, 0.01%, or 0.001 %) of anti-foam selected from different commercially available preparations from Dow Corning (Anti-foam AF, FG-10) or Sigma (SE-15) and different amounts of target DNA. Results are summarized in Table 4.

The methods and compositions of this invention may be used for a variety of enzymatic reactions, and it has been found that DNA polymerase reactions are compatible with addition of anti foam agents. Unexpectedly, the activity of reverse transcriptases, such as MMLV RT, also is compatible with anti foam agents and the method of invention has been used for RT PCR in amplification of RNA. It will be apparent to those skilled in the art that different concentrations or combinations of anti-foam agents may be used for various PCR formulations. Although some anti-foam agents have an adverse effect on PCR activity or efficiency due to inhibition of enzyme activity, they still can be used for some applications, as described below.

Surprisingly, the methods and compositions described in this invention are also compatible with use of antibodies. The reagents used in examples IV and V contained anti-Taq DNA polymerase antibodies resulting in antibody mediated "hotstart" PCR reaction. The present invention method and use of anti-foam agents can therefore be used for a variety of antibody-based immunoassays and other protocols involving protein-protein or protein-ligand interactions. Examples of these types of assays include, but are not limited to, protein chips and antibody chips.

The methods and the compositions of the invention can also be used in preparation of lyophilized or dried reagents for use in enzymatic or protein binding assays. There are a number methods known in the art for stably maintaining enzymes, other proteins and other reaction components in dry form that can be reconstituted for use. The dry preparations are especially useful in diagnostic methods and kits. Use of agents and improvements in the accuracy of results and the reactions provide significant advantages for diagnostic procedures involving reaction components with detergents.

The methods of the present invention are also applicable to in vitro transcription reactions with RNA polymerases. As shown in example VII presence of antifoam agents are compatible with T7 RNA polymerase and in vitro transcription reaction. Unexpectedly, the presence of antifoam agent improved the kinetics of transcription by T7 RNA polymerase compared to the control reactions without the antifoam agents.

Until the present invention, the effects of various antifoam agents on enzymatic reactions was generally unknown, and their use has been limited to hybridization reactions and experimental conditions where enzymes are not used or their activity is not required. Two references relating to the inclusion of antifoam compounds in enzymatic reactions are in US patent Nos. 5,985,569 and 5,962,273. In both of these references an unspecified anti foam agent was used in an isothermal strand displacement amplification reaction for detection of bacterial sequences. The reactions were performed at 52.6°C and did not involve thermocycling between high (>80°C ) and low (<60°C) temperatures. No reason was provided as to why the unidentified antifoam reagent was included in the reaction, nor was any effect on the reaction recorded. Neither reference suggests the use of antifoams in thermocycling reactions or in high temperature reactions. Similarly, neither reference recognizes the problems associated with fluid handling and optical monitoring in thermocycling and high temperature reactions.

The present inventors have studied a variety of different antifoam compounds and demonstrated that different antifoams affect various enzymatic reactions differently. The present invention provides preferred compositions containing antifoams that may be used in real time PCR assays and that demonstrate significant improvements in optical detection of real time PCR. In addition, the addition of antifoam improves the liquid handling and accuracy of liquid handling in high-throughput reactions and automated settings.

Optimal conditions and compositions for use of antifoam agents in various enzymatic reactions are described. The reactions used include, but are not limited to, the polymerase chain reaction using a thermostable DNA polymerase such as Taq, and reverse transcription of RNA into cDNA using a variety of reverse transcriptases. Methods are describe that permit use of two-subunit RT's (such as AMV) and single subunit RT's (MMLV). Methods of using anti foam agents during *in vitro* transcription of RNA also are described. These methods permit more efficient amplification reactions and provide an improved method for quantitation of gene expression.

The present inventors also demonstrated that some antifoams, when used in high concentrations (>0.01 %, example I) imparted a cloudy appearance that interfered with optical detection. Notably, both US patent Nos. 5,985,569 and 5,962,273 employed the (unidentified ) antifoam reagent at relatively high concentrations (0.015% and 0.019%). Example V below shows that anti foam concentrations of 0.1 % and greater were inhibitory to PCR.

**Use of anti-foam agents in enzymatic reactions.**

Preparation of anti-foam containing PCR reactions can be done in a variety of ways. For example, anti-foam agents can be added to any one of the buffers used for PCR prior to or at the time of reaction assembly. Anti-foam agents can also be added to premixed PCR formulations such as Mastermixes and kept stably under usual storage conditions for PCR reactions. In certain embodiments of the present invention anti-foam containing mastermixes may advantageously be used directly with automated liquid handling devices such as robots and microfluidic devices.

Throughout this disclosure, various terms that are generally understood by those of routine skill in the art are used. The skilled artisan will appreciate, for example, that the term "dNTP" (plural "dNTPs") generically refers to the deoxynucleoside triphosphates (e.g., dATP, dCTP, dGTP, dTTP, dUTP, dITP, 7-deaza-dGTP, adATP, adTTP, adGTP and adCTP), and the term "ddNTP" (plural "ddNTs") to their dideoxy counterparts, that are incorporated by polymerase enzymes into newly synthesized nucleic acids.

The term "unit" as used herein refers to the activity of an enzyme. When referring to a thermostable DNA polymerase, one unit of activity is the amount of enzyme that will incorporate 10 nanomoles of dNTPs into acid-insoluble material (i.e., DNA or RNA) in 30 minutes under standard primed DNA synthesis conditions.

"Working concentration" is used in the context of the present invention to mean the concentration of a reagent that is at or near the optimal concentration used in a solution to perform a particular function (such as amplification, sequencing or digestion of nucleic acids).

The term "detergent" in the present context refers to detergent compositions that generally are added to PCR and RT-PCR to improve reaction performance by, for example, stabilizing a polymerase in the reaction mixture. Examples of detergents used include nonionic surfactants such as TRITON X-100, Nonidet P-40 (NP-40), Tween 20 or Brij 35. The skilled artisan will recognize that other nonionic surfactants are known in the art and may be used in the methods and compositions of the invention.

In the context of the present invention, an "effective amount" of an anti-foam agent in a reaction mixture is an amount or concentration that suppresses foaming/bubble formation to an extent necessary to permit accurate optical analysis of the reaction mixture or accurate fluid handling, especially of small volumes of reaction mixture, but that does not substantially inhibit enzyme activity in the reaction mixture. In the context of the present invention, enzyme activity in a reaction mixture is substantially inhibited when the enzyme no longer functions adequately to achieve the desired purpose of carrying out the reaction. For example, in a quantitative PCR reaction using a defined number of heating/cooling cycles, substantial inhibition of the enzymatic activity in the reaction can result in a reduction of the amount of product that is insufficient to be accurately quantified. Alternatively, the enzyme activity could be substantially inhibited by a reduction in the accuracy of the enzymatic reaction (for example, by incorporation of non-complementary dNTPs during a primer-dependent polymerase reaction, or by mis-priming) such that the identity of the reaction product is compromised.

The present invention provides, in a first preferred embodiment, compositions comprising mixtures of one or more anti-foam reagents, one or more detergents, one or more thermostable enzyme (e.g., a thermostable DNA polymerase, restriction enzyme, etc.), one or more buffer salt, and other reagents necessary for carrying out the procedure associated with the enzyme(s) (e.g., deoxynucleoside triphosphates (dNTPs) for amplification of nucleic acids, dNTPs and dideoxynucleoside triphosphates (ddNTPs) for sequencing of nucleic acids, etc.). In additional embodiments, the invention provides compositions that further comprise one or more moieties, such as antibodies, that specifically bind to the one or more thermostable enzymes (such as the one or more DNA polymerases) in the compositions. The compositions of the invention also may include other stabilizing compounds (e.g., glycerol, serum albumin or gelatin) that traditionally have been included in stock reagent solutions for enzymes. Furthermore, the invention provides these reagent compositions in ready-to-use concentrations, obviating the time-consuming dilution and pre-mixing steps necessary with previously available solutions. Unexpectedly, even at these diluted concentrations the reagent compositions are stable for extended periods of time at temperatures ranging from ambient (about 20-25° C.) to about -70° C.

The agents of the invention can be dissolved in water or other appropriate solvents and mixed in desired concentration with any of the components required for reaction assembly. The buffer mix used for PCR reactions may advantageously be used. As is evident to those skilled in the art, the anti-foam agents can be added directly or can be mixed with at least one of the components necessary for the desired reaction. In additional embodiments, the present invention provides these ready-to-use compositions in the form of kits that are suitable for immediate use to carry out the procedure associated with the enzyme(s) (e.g. nucleic acid amplification or sequencing in the case of DNA polymerases). These kits are also stable for extended periods of time at temperatures ranging from ambient (about 20-25° C.) to -70° C.

In additional embodiments, the invention provides ready-to-use compositions for PCR amplification. The ready-to-use reagents contain all necessary components for PCR amplification such as one or more DNA polymerase(s), one or more deoxynucleoside triphosphates (dNTPs) and buffers, and optionally one or more other components contributing to efficient amplification of nucleic acid templates by automatic "hot start." Automatic Hot Start PCR can be accomplished by reaction of specific antibodies, e.g., monoclonal antibodies, that bind to and inactivate one or more DNA polymerases, such as thermostable DNA polymerases (e.g., Taq DNA polymerase), that are present in the ready-to-use compositions of the invention. In additional embodiments, the invention provides formulation of ready-to-use PCR reagents that contain one or more thermostable DNA polymerases (e.g., Taq DNA polymerase), one or more dNTPs, one or more buffers, and one or more specific binding moieties, such as antibodies, that bind to a DNA polymerase.

**Sources of Reagents**

The compositions of the present invention may be formed by mixing the component reagents at the concentrations described below. The components for making the ready-to-use compositions can be obtained from, for example, Invitrogen (Carlsbad, CA).

Thermostable Enzymes

The thermostable enzymes (e.g., DNA polymerases, restriction enzymes, phosphatases, etc.) used in the present invention may be isolated from natural or recombinant sources, by techniques that are well-known in the art (See Bej and Mahbubani, Id.; WO 92/06200; WO 96/10640), from a variety of thermophilic bacteria that are available commercially (for example, from American Type Culture Collection, Manasas, VA.) or may be obtained by recombinant DNA techniques (WO 96/10640). Suitable for use as sources of thermostable enzymes or the genes thereof for expression in recombinant systems are the thermophilic bacteria Thermus thermophilus, Thermococcus litoralis, Pyrococcus furiosus, Pyrococcus woosii and other species of the Pyrococcus genus, Bacillus sterothennophilus, Sulfolobus acidocaldarius, Thermoplasma acidophilum, Thermus flavus, Thermus ruber, Thermus brockianus, Thermotoga neapolitana, Thermotoga maritima and other species of the Thennotoga genus, and Methanobacterium thermoautotrophicum, and mutants thereof. It is to be understood, however, that thermostable enzymes from other organisms may also be used in the present invention without departing from the scope or preferred embodiments thereof. As an alternative to isolation, thermostable enzymes (e.g., DNA polymerases) are available commercially from, for example Invitrogen (Carlsbad, CA), New England Biolabs (Beverly, Mass.), Finnzymes Oy (Espoo, Finland) and Applied Biosystems (Foster city, CA). Once obtained, the purified enzymes may be placed into solution at working concentrations and stored according to the methods of the present invention.

dNTPs

The dNTP components of the present compositions serve as the "building blocks" for newly synthesized nucleic acids, being incorporated therein by the action of the polymerases. These dNTPs--deoxyadenosine triphosphate (dATP), deoxycytosine triphosphate (dCTP), deoxyguanosine triphosphate (dGTP), deoxythymidine triphosphate (dTTP), and for some applications deoxyuridine triphosphate (dUTP) and deoxyinosine triphosphate (dITm), a-thio-dATP and 7-deaza-dGTP--are available commercially from sources including Invitrogen (Carlsbad, CA), New England Biolabs (Beverly, Mass.) and Sigma Chemical Company (Saint Louis, Mo.). The dNTPs may be unlabeled, or they may be detectably labeled by coupling them by methods known in the art with radioisotopes (e.g., $^3$H, $^{14}$C, $^{32}$P or $^{35}$S), vitamins (e.g., biotin), fluorescent moieties (e.g., fluorescein, rhodamine, Texas Red, or phycoerythrin) or other detection agents. Labeled dNTPs may also be obtained commercially, for example Invitrogen (Carlsbad, CA) or Sigma Chemical Company (Saint Louis, Mo.). Once obtained, the dNTPs may be placed into solution at working concentrations and stored according to the methods of the present invention.

ddNTPs

The ddNTP components of the present compositions serve as the "terminating agents" in the dideoxy nucleic acid sequencing methodologies, being incorporated into newly synthesized nucleic acids by the action of the polymerases. These ddNTPs—dideoxyadenosine triphosphate (ddATP), dideoxycytosine triphosphate (ddCTP), dideoxyguanosine triphosphate (ddGTP), dideoxythymidine triphosphate (ddTTP), and for some applications dideoxyuridine triphosphate (ddUTP) and dideoxyinosine triphosphate (ddITP)-are available commercially from sources including Invitrogen (Carlsbad, CA), New England Biolabs (Beverly, Mass.) and Sigma Chemical Company (Saint Louis, Mo.). The ddNTPs may be unlabeled, or they may be detectably labeled by coupling them by methods known in the art with radioisotopes (e.g., $^3$H, $^{14}$C, $^{32}$P, or $^{35}$S), vitamins (e.g., biotin), fluorescent moieties (e.g., fluorescein, rhodamine, Texas Red, or phycoerythrin) or other detection agents. Labeled ddNTPs may also be obtained commercially, for example from Invitrogen, Inc. (Carlsbad, Md.) or Sigma Chemical Company (Saint Louis, Mo.). Once obtained, the ddNTPs may be placed into solution at working concentrations and stored according to the methods of the present invention.

Buffers/Salts

All buffers and cofactor salts comprising the compositions of the present invention, and concentrated stock solutions thereof are available from a variety of commercial sources including Invitrogen (Carlsbad, CA) and Sigma Chemical Company (Saint Louis, Mo.). Particularly preferred buffers for use in forming the present compositions are the sulfate, hydrochloride, phosphate or free acid forms of tris-(hydroxymethyl)aminomethane (TRIS®), although alternative buffers of the same approximate ionic strength and pKa as TRIS® may be used with equivalent results. In addition to the buffer salts, cofactor salts such as those of potassium (preferably potassium chloride) and magnesium (preferably magnesium chloride or sulfate) are included in the compositions. Once obtained, the buffers and cofactor salts may be placed into solution at working concentrations and stored according to the methods of the present invention.

Detergents

At least one detergent may be included as a component of the present compositions, to provide for both increased stability and activity of the component enzymes. Nonionic detergents are preferred, to maintain a balanced ionic strength and prevent chelation of cofactors and aggregation or inactivation of proteins. Particularly preferred as detergents are TRITONX-100®., Brij 35, Tween20 and Nonidet P-40 (NP-40), although other nonionic surfactants and mixtures thereof may also be used in the present compositions. These detergents are available commercially from sources such as Sigma Chemical Company (Saint Louis, Mo.), usually as concentrated aqueous solutions or in powder form. Once obtained, the detergents may be placed into solution at working concentrations and stored according to the methods of the present invention.

Binding Moieties

In additional embodiments of the invention, the compositions may optionally comprise one or more specific binding moieties, such as antibodies, that specifically bind to the one or more thermostable enzymes, such as the one or more DNA polymerases, present in the compositions of the invention. According to this aspect of the invention, the one or more binding moieties will specifically bind to the one or more thermostable enzymes (such as the one or more DNA polymerases) at temperatures below about 45° C.; as a result of this binding, the enzymatic activity of the enzyme will be completely or substantially completely inhibited. However, once the composition or reaction mixture containing the composition is raised to a temperature above about 60-65.° C. (e.g., the temperatures at which standard PCR methods are conducted), the antibody is denatured and the activity of the enzyme is restored. Thus, such compositions will have utility in such applications as "Hot Start" PCR amplification protocols. Antibodies for use in this aspect of the invention include polyclonal antibodies, monoclonal antibodies, and enzyme-binding fragments (such as F (ab') or F(ab')$_2$ fragments) thereof. Any binding moiety, such as an antibody or fragment thereof, which specifically binds to one or more of the thermostable enzymes in the present compositions, such as the DNA polymerases, may be used, including but not limited to anti-Taq antibodies, anti-Tne antibodies, anti-Tma antibodies, anti-Pfu antibodies, anti-Pwo antibodies, anti-Tth antibodies, and the like. These and other antibodies suitable for use in this aspect of the invention may be obtained commercially, e.g., from Invitrogen. (Carlsbad, CA). Alternatively, antibodies may be produced in animals by routine methods of production of polyclonal antibodies (see, e.g., Harlow, E., and Lane, D., Antibodies: A Laboratory Manual, Cold Spring Harbor, N.Y.: Cold Spring Harbor Laboratory Press (1988); Kaufinan, P. B., et al., In: Handbook of Molecular and Cellular Methods in Biology and Medicine, Boca Raton, Fla.: CRC Press, pp. 468-469 (1995) or monoclonal antibodies (see, e.g., Kohler et al., Nature 256:495 (1975); Kohler et al., Eur. J Immunol. 6:511 (1976); Kohler et al., Eur. J. Immunol. 6:292 (1976); Hammerling et al., In: Monoclonal Antibodies and T-Cell Hybridomas, New York: Elsevier, pp. 563-681 (1981); Kaufinan, P. B., et al., In: Handbook of Molecular and Cellular Methods in Biology and Medicine, Boca Raton, Fla.: CRC Press, pp. 444-467 (1995)), using the corresponding thermostable enzyme (such as the corresponding DNA polymerase) as an immunogen.

Antifoam

Foam control agents are chemicals or formulated products additives that degas or deaerate foam in liquid media. They are thought to act by reducing bubble surface tension or by penetrating the bubble wall and destabilizing the liquid-gas interface causing the bubble to collapse. Such agents have been used in a wide variety of consumer and industrial applications such as personal care products, foods medicine, release agents, antifoams and dielectric fluids but, prior to the present application, had not been used in PCR reactions. Foam control agents can be classified as either defoamers, which act to break up previously formed foam, or antifoams, which act to prevent the formation of foam. Often these terms are used interchangeably for similar compounds depending on the point in the process were the chemical is applied. Depending on the particular application, a wide variety of chemicals can function as antifoams. While the composition of commercially available antifoam is generally proprietary, formulations of fatty acid esters, or emulsions of silicon fluids, such as simethicone, polydimethylsiloxanes, octamethylcyclotetrasiloxane are widely used in food processing, pharmaceutical, and bioprocessing industries. Examples of available antifoam agents from DOW Corning for medical/pharmaceutical applications include, but are not limited to mixtures of polydimethylsiloxane fluid and silica, such as Q7-2243 LVA and Antifoam M, Q7-2587, or water soluble, nonionic emulsion of 30% simethicone, such as 7-9245 and Medical Antifoam C. Examples of food grade silicone emulsions from DOW Coming include FG-10 Emulsion, Antifoam H-10 Emulsion, 1510-US Emulsion, 1520-US Emulsion, Antifoam A Compound, Antifoam AF Emulsion, and Antifoam C Emulsion. Sigma Chemical Company provides a number of proprietary antifoam formulations for cell culture applications. These include Nonsilicone polypropylene based polyether formulations, such as A6426 and Antifoam 204, other organic antifoams such as O-10 or O-60, fatty-acid ester type antifoam O-30, and numerous silicone based polymer emulsions and concentrates (Antifoam A, Antifoam B Emulsion, Antifoam C Emulsion, SO-25, SE-15, or SE-35) as well as mixtures of organic and silicone antifoams (Antifoam 289).

One skilled in the art will recognize that selection of an anti-foam reagent and choice of appropriate concentration for in vitro enzymatic reactions as described in the present invention may require some routine experimentation. Such experimentation would involve, for example, comparing results in reactions that are identical except for the identity and concentration of anti-foam reagent used. Such simple experiments will indicate both the identity and suitable concentration of an anti-foam reagent for any given application. Effective concentrations of the anti-foam reagent can vary from 0.0001% to 10%, although many reagents will work optimally in the range of 0.0001-0.1%. As shown in the examples set forth below, the optimal concentration of antifoam varies for various applications. This is also dependent on the concentration of detergents used in various applications. It may be necessary to optimize, by routine screening of the type described above, the concentration of antifoam depending on the detergent and its concentration.

Formulating Reagent Compositions

Once the reagent components are obtained, they are mixed at working concentrations to form a solution suitable for immediate use with or without dilution or addition of further reagents. The water used in the formulations of the present invention is preferably distilled, deionized and sterile filtered (through a 0.1-0.2 micrometer filter), and is free of contamination by DNase and RNase enzymes. Such water is available commercially, for example from Sigma Chemical Company (Saint Louis, Mo.), or may be made as needed according to methods well known to those skilled in the art.

Although the components of the present compositions may be admixed in any sequence, it is often preferable to first dissolve the buffer(s) and cofactor salts in water and to adjust the pH of the solution prior to addition of the remaining components. In this way, the pH-sensitive components (particularly the enzymes, ddNTPs and dNTPs) will be less subject to acid- or alkaline-hydrolysis during formulation.

To formulate the buffered salts solution, a buffer salt which is preferably a salt of tris(hydroxymethyl)aminomethane (TRIS.®), and most preferably the hydrochloride salt thereof, is combined with a sufficient quantity of water to yield a solution having a TRIS® concentration of 5-150 mM, preferably 10-60 .mM, and most preferably about 20-60 mM. To this solution, a salt of magnesium (preferably either the chloride or sulfate salt thereof) may be added to provide a working concentration thereof of 1-10 millimolar, preferably 1.5-5 mM, and most preferably about 1.5-2 mM. A salt of potassium (most preferably potassium chloride) may also be added to the solution, at a working concentration of 10-100 mM and most preferably about 50 mM. An ammonium salt, for example ammonium sulfate, may also be added to the mixture, at a working concentration of 2-50 mM, preferably 10-30 mM and most preferably 18 mM. Combinations of ammonium sulfate and potassium chloride (or other salts) may also be used in formulating the compositions of the present invention. A small amount of a salt of ethylenediaminetetraacetate (EDTA) may also be added (preferably about 0.1 mM), although inclusion of EDTA does not appear to be essential to the function or stability of the compositions of the present invention. After addition of all buffers and salts, this buffered salt solution is mixed well until all salts are dissolved, and the pH is adjusted using methods known in the art to a pH value of 7.4 to 9.2, preferably 8.0 to 9.0, and most preferably about 8.3 for compositions to be used in amplification or sequencing of nucleotide fragments up to about 5-6 kilobases in size (hereinafter referred to as "standard compositions"), and about 8.9 for compositions to be used for amplification or sequencing of nucleotide fragments larger than about 5-6 kilobases in size (hereinafter referred to as "large sequence compositions").

To the buffered salt solution, the remaining components of the present composition are added. It is well known in the field that the addition of one or more detergents to an aqueous buffer will aid in the subsequent solubilization of added proteins. Accordingly, at least one nonionic detergent such as TRITON X-100® (preferably at a working concentration of 0.1-1%), Brij 35 (preferably at a concentration of 0.01-1% and most preferably of about 0.1%) or Nonidet P-40 (NP-40, preferably as an admixture with a concentration of 0.004-1 %, and most - preferably in admixture with Tween 20 at a working concentration of 0.1% for standard compositions and 0.02% for large sequence compositions) maybe added to the buffer solution. This detergent is preferably added prior to the introduction of the remaining components into the solution, although the detergent may equivalently be added at any step of formulation. Following formulation, the buffered salt solutions may be filtered through a low protein-binding filter unit that is available commercially (for example from Millipore Corporation, Bedford, Mass.) and stored until use.

The remaining components are then added to the solution to formulate the compositions of the present invention. At least one thermostable enzyme (e.g., DNA polymerase) is added and the solution is gently mixed (to minimize protein denaturation). For standard DNA amplification (including via PCR) or sequencing of DNA segments up to about 5-6 kilobases in length, any thermostable DNA polymerase (hereinafter the "primary polymerase") may be used in the standard compositions, although Taq, Tne, Tma, VENT™, DEEPVENT.™, Pfu or Pwo polymerases are preferable at a working concentration in the solution of about 0.1-200 units per milliliter, about 0.1-50 units per milliliter, about 0.1-40 units per milliliter, about 0.1-36 units per milliliter, about 0.1-34 units per milliliter, about 0.1-32 units per milliliter, about 0.1-30 units per milliliter, or about 0.1-20 units per milliliter, and most preferably at a working concentration of about 20 units per milliliter. For amplification of DNA segments larger than 5-6 kilobases in length, large sequence compositions should be formulated by adding to the standard compositions a low concentration of one or more additional thermostable DNA polymerases (hereinafter the "secondary polymerase") containing a 3'-5' exonuclease activity. Particularly suited for this application are VENT™, Pfu, Pwo or Tne, and most preferably DEEPVENT™, DNA polymerases. The additional polymerase(s) should be added to the solution in sufficient quantity to give a final working concentration of about 0.0002-200 units per milliliter, about 0.002-100 units per milliliter, about 0.002-20 units per milliliter, about 0.002-2.0 units per milliliter, about 0.002-1.6 units per milliliter, about 0.002-0.8 units per milliliter, about 0.002-0.4 units per milliliter, or about 0.002-0.2 units per milliliter, most preferably at concentrations of about 0.40 units per milliliter.

It has heretofore been thought that the activity ratios of the primary to secondary polymerases should be maintained at about 4:1-2000:1 for large sequence amplification (see U.S. Pat. No. 5,436,149). It has now been discovered, however, that in the compositions of the present invention that activity ratios of the primary to secondary polymerases of 1:1, 1:2, 1:4, 1:5, 1:8, 1:10, 1:25, 1:50, 1:100, 1:250, 1:500, 1:1000 and 1:2000 often may be suitable for amplification of large nucleotide sequences.

For nucleic acid sequencing, the reagent compositions may be used as formulated above. For nucleic acid sequencing by the dideoxy method (See U.S. Pat. Nos. 4,962,020, 5,173,411 and 5,498,523), however, preferably the mutant Tne DNA polymerase is added to the reagent compositions. Tne polymerase is added to the solution to give a working concentration of about 0.1-10,000 units per milliliter, about 0.1-5000 units per milliliter, about 0.1-2500 units per milliliter, about 0.1-2000 units per milliliter, about 0.1-1500 units per milliliter, about 0.1-1000 units per milliliter, about 0.1-500 units per milliliter, about 0.1-300 units per milliliter, about 0.1-200 units per milliliter, about 0.1-100 units per milliliter, or about 0.1-50 units per milliliter, and most preferably of about 300 units per milliliter.

For dideoxy sequencing, a solution of each ddNTP is also prepared. The base of each solution contains dATP, dCTP, dTTP, 7-deaza-GTP and/or other dNTPs, each at a working concentration of about 10-1000 $\mu$M, about 10-500 $\mu$m, about 10-250 $\mu$M, or about 10-100 $\mu$M, most preferably at a concentration of about 100 $\mu$M, in a solution of buffer and chelating salts, for example TRIS®.-HCl most preferably at a working concentration of about 10 mM (pH about 7.5) and disodium-EDTA most preferably at a concentration of about 0.1 mM. To this base, one of the ddNT's is added to make each of four solutions. Preferably, the sodium or lithium salt of ddATP, ddCTP, ddGTP or ddTTP is added to the solution to give a working concentration of the ddNTP of about 0.5-10 $\mu$M, about 0.5-8 $\mu$M, about 0.5-5 $\mu$M, about 0.5-3 $\mu$M, about 0.5-2.5 $\mu$M, or about 0.5-2 $\mu$M, and most preferably about 2 $\mu$M. For cycle sequencing applications, the pH of the ddNTP solutions will preferably be about 9.0, and the concentrations of ddNTPs may be lower, preferably about 0.05 to 1.0 $\mu$M or about 0.05 to 0.8 $\mu$M, and most preferably about 0.08 to 0.8 $\mu$M. For some applications, it may be desirable to also incorporate or substitute ddITP, ddUTP, and/or .alpha.-thio-dATP into the compositions at approximately the same working concentrations. Thus, four solutions are prepared, each containing one of the four ddNTPs, which are combined with the polymerase compositions of the present invention to carry out the four separate reactions used in dideoxy sequencing. Alternatively, for single-solution sequencing as disclosed in U.S. Pat. Nos. 4,962,020 and 5,173,411, the four ddNTPs may be combined into a single solution which is added to the polymerase compositions of the present invention to perform the sequencing reaction.

For nucleic acid amplification, including PCR, dNTP salts are added to the reagent compositions. Preferably, the sodium or lithium salts of dATP, dCTP, dGTP and dTTP are added to the solution to give a working concentration of each dNTP of 10-1000 $\mu$M, preferably 200-300 $\mu$M, and most preferably about 200 $\mu$M. For some applications, it may be desirable to also incorporate or substitute dITP or dUTP into the compositions at the same working concentrations.

In certain embodiments as noted above, one or more antibodies that specifically bind to the one or more thermostable enzymes in the compositions, such as the one or more DNA polymerases, may optionally be added to the compositions. Preferably, the antibodies are used in these compositions at an antibody to polymerase concentration ratio of up to about 100:1, up to about 50:1, up to about 25:1, up to about 20:1, up to about 15:1, up to about 10:1, up to about 9:1, up to about 8:1, up to about 7.5:1, up to about 7:1, up to about 6:1, up to about 5:1, up to about 4:1, up to about 3:1, up to about 2.5:1, up to about 2:1, or up to about 1:1. Most preferably, the antibodies are used in the compositions at an antibody to polymerase concentration ratio of about 1:1 to about 10:1, or about 1:1 to about 5:1.

To reduce component denaturation, the reagent compositions preferably are stored in conditions of diminished light, e.g., in amber or otherwise opaque containers or in storage areas with controlled low lighting. The ready-to-use reagent compositions of the present invention are unexpectedly stable at ambient temperature (about 20°-25°C.) for about 4-10 weeks, are stable for at least one year upon storage at 4°C., and for at least two years upon storage at -20°C. Surprisingly, storage of the compositions at temperatures below freezing (e.g., - 20°C. to -70°C.), as is conventional with stock solutions of bioactive components, is not necessary to maintain the stability of the compositions of the present invention.

In other preferred embodiments, the compositions of the present invention may be assembled into kits for use in nucleic acid amplification or sequencing. Sequencing kits according to the present invention comprise a carrier means, such as a box, carton, tube or the like, having in close confinement therein one or more container means, such as vials, tubes, ampoules, bottles and the like, wherein a first container means contains a stable composition comprising a mixture of reagents, at working concentrations, which are at least one thermostable DNA polymerase, at least one buffer salt, at least one deoxynucleoside triphosphate, at least one dideoxynucleoside triphosphate, and optionally at least one antibody which specifically binds to at least one thermostable DNA polymerase present in the compositions. The sequencing kits may further comprise additional reagents and compounds necessary for carrying out standard nucleic sequencing protocols, such as pyrophosphatase, agarose or polyacrylamide media for formulating sequencing gels, and other components necessary for detection of sequenced nucleic acids (See U.S. Pat. Nos. 4,962,020 and 5,498,523, which are directed to methods of DNA sequencing).

**EXAMPLE I: Real-time TaqMan PCR in the presence of varying amounts of anti-foam.**

This example demonstrates the ability of PCR to proceed in the presence of an anti-foam compound.

Real-time quantitative polymerase chain reactions specific for the human cytoplasmic β-actin sequence were carried out using a commercial hot-start *Taq* DNA polymerase reaction cocktail as follows. Each 50 μl PCR contained 1X iQ PCR SuperMix (Bio-Rad Laboratories), 200 nM each primer, and 100 nM FAM and TAMRA labeled 5'-nuclease probe as described by Xu et al. 2000, Focus 22:3-5 (forward primer: 5'-CCTGGCACCCAGCACAAT-3'; reverse primer: 5'-GGGCCGGACTCGTCATAC-3'; Taqman probe: 5'-FAM- AGCCGCCGATCCACACGGAGT- TAMRA- 3'), and varying amounts of a DNA target. Triplicate PCRs were performed for each amount of input DNA ($1 \times 10^2$, $1 \times 10^4$, $1 \times 10^6$, or $1 \times 10^8$ copies of a plasmid containing the gene encoding human cytoplasmic β-actin). PCRs were conducted under identical conditions except for the inclusion of varying amounts (0. 1 %, 0.01 %, 0.001 %, 0.0001 %, or 0) of anti-foam 1520-US from Dow Coming.

Reactions were assembled at room temperature in 96-well PCR plates, sealed with optically clear heat-seal film (Marsh Bioproducts), and temperature cycled using a Bio-Rad iCycler optical thermal cycler. PCRs were incubated at 95°C for 3 min followed by 45 cycles of 95°C, 15s; 60°C, 45s. Fluorescence signal was monitored during the annealing/extension step and analyzed using the accompanying iCycler software. Cycle threshold (Ct) values for each PCR were determined using baseline-normalized fluorescence signal (cycles 2 to 11) and a constant threshold fluorescence (75 RFU) for each run. Results are summarized in table 1.

Results demonstrated that the inclusion of anti-foam in PCR does not inhibit the kinetics of DNA amplification or interfere with optical detection of the 5'-nuclease TaqMan assay. Linear regression analysis of the log of DNA copy input versus Ct number indicated that inclusion of anti-foam at all concentrations that were tested, improved PCR efficiency. Concentrations of as low as 0.001%, were sufficient to eliminate bubbles in the reaction cocktail and improve its liquid handling properties. However, anti-foam concentrations of 0.1% or 0.01% imparted a cloudy appearance to the reaction. Therefore, optimal concentration of a given anti-foam compound must be determined empirically and is a balance between the cloud point, the efficacy of the compound to eliminate bubbles from surfactants, and any potential adverse effect on DNA amplification.

**EXAMPLE II: Real-time TaqMan PCR in the presence of different anti-foam compounds.**

This example demonstrates the ability of PCR to proceed in the presence of a variety of anti-foam compounds.

Real-time quantitative polymerase chain reactions specific for the human cytoplasmic β-actin sequence were carried out as described above in example 1. PCRs were conducted under identical conditions except for the inclusion of 0.005% of anti-foam selected from different commercially available preparations from Dow Corning (1520-US, AF, or FG-10) or Sigma (O-30, SE-15, or Antifoam B). Control reactions omitted anti-foam. Results are summarized in table 2.

Results demonstrated that a variety of anti-foams with different chemical compositions, fatty acid ester (Sigma O-30), or silicone emulsions comprised of polydimethylsiloxane, and emulsion formulations are effective at suppressing foaming by PCR surfactants without adverse effect on DNA amplification. For all anti-foams tested, the presence of 0.005% anti-foam improved liquid handling properties, optical clarity and PCR efficiency relative to control reactions that omitted anti-foam.

## EXAMPLE III: Real-time SYBR Green I PCR in the presence of antifoam reagent

This example extends the efficacy and compatibility of anti-foam in real-time PCR using different homogeneous detection chemistries, specifically, fluorescent dsDNA-specific dyes.

Real-time quantitative polymerase chain reactions specific for the human cytoplasmic βactin sequence were carried out using a commercial hot-start *Taq* DNA polymerase reaction cocktail modified for use with SYBR Green I as follows. Each 50 $\mu$l PCR contained 1X iQ PCR SuperMix (Bio-Rad Laboratories), 2% DMSO, 0.25X SYBR Green I (Molecular Probes), 300 nM each primer, as described by Xu et al. 2000, Focus 22:3-5 (forward primer: 5'-CCTGGCACCCAGCACAAT-3'; reverse primer: 5'-GGGCCGGACTCGTCATAC-3'), and varying amounts of a DNA target. Triplicate PCRs were performed for each amount of input DNA ($1\times10^2$, $1\times10^4$, $1\times10^6$, or $1\times10^8$ copies of a plasmid containing the gene encoding human cytoplasmic β-actin). PCRs were conducted under identical conditions except for the inclusion of 0.005% of anti-foam selected from different commercially available preparations from Dow Coming (1520-US, AF, or FG-10) or Sigma (O-30, SE-15, or Antifoam B). Control reactions omitted anti-foam.

Reactions were assembled at room temperature in 96-well PCR plates, sealed with optically clear heat-seal film (Marsh Bioproducts), and temperature cycled using a Bio-Rad iCycler optical thermal cycler. PCRs were incubated at 95°C for 3 min followed by 45 cycles of 95°C, 15s; 60°C, 20s; 68°C, 20s. Fluorescence signal was monitored during the 68°C extension step and analyzed using the accompanying iCycler software. Cycle threshold (Ct) values for each PCR were determined using baseline-normalized fluorescence signal (cycles 2 to 10) and a constant threshold fluorescence (100 RFU) for each run. Results are summarized in table 3.

Results demonstrated that a variety of anti-foams with different chemical compositions, fatty acid ester (Sigma O-30) or silicone emulsions are effective at suppressing foaming by PCR surfactants without adverse effect on PCR efficiency in the presence of SYBR Green I PCR. For all anti-foams tested, the presence of 0.005% anti-foam improved liquid handling properties, optical clarity and PCR efficiency relative to control reactions that omitted anti-foam. Mean Ct values for 100 copy PCRs containing either Dow 1520-US or Sigma O-30 were 1 cycle lower than control reactions lacking. These data indicate that the use of anti-foam 1520-US or O-30 can improve the efficacy of SYBR Green I PCR for amplification of low copy nucleic acid analytes.

## EXAMPLE IV: Inclusion of anti-foam compound improves the precision and reliability of low-copy quantitative PCR.

Real-time quantitative polymerase chain reactions specific for the human cytoplasmic β-actin sequence were carried out as essentially described above in example 3. A mastermix that was sufficient for 110, 50-$\mu$l reactions was prepared containing 1X iQ PCR SuperMix (Bio-Rad Laboratories), 2% DMSO, 0.25X SYBR Green I (Molecular Probes), 300 nM each primer, and 20 copies of β-actin DNA template for each 50-$\mu$l reaction. This was divided into equal aliquots. Dow 1520-US anti-foam was added to a final concentration of 0.003% to one aliquot and an equivalent volume of water was added to the other. 50-$\mu$l aliquots from either mastermix were dispensed into each of 48 wells of a 96 well PCR plate using a multi-dispensing digital pipettor (Rainin). The plate was sealed as described in example 1 and cycled as described in example 3.

The average of Ct results for control reactions was 33.41 with a standard deviation of 1.29. The average of Ct results for reactions containing anti-foam was 32.62 with a standard deviation of 0.87. These data support the conclusion that addition of anti-foam improved precision and sensitivity of low copy PCR. Additionally, optical interference from bubbles in a limited number of control reactions required manual intervention in determining the best cycle range to use for baseline normalization of fluorescent signal (cycles 15 to 30) to achieve optimal Ct results for the control PCRs. Bubbles were absent in reactions that contained anti-foam.

Ideally, the basal fluorescence of a real-time reaction should be invariant from cycle to cycle until the accumulation of PCR product is sufficient to produce signal above background. Surfactants present in PCR and *Taq* DNA polymerase preparations, however, can result in bubbles when reactants are mixed. Failure to clear reactions of bubbles prior to PCR cycling can distort optical signals and skew background fluorescence readings. This effect is illustrated in the amplification plots for wells H2 and H5 presented in figures 1 and 2. A pronounced increase in fluorescence is visible near cycle 5 for well H2 and cycle 11 for well H5 (figure 1). The amplification plots presented in figure 2 demonstrate the effect of these fluorescent perturbations on Ct determination when they are included in the data set used to normalize all PCRs to a common baseline. Under these circumstances, the average Ct for these 6 reactions was 35.99 with a standard deviation of 2.19. In contrast, the 6 PCRs that contained anti-foam (figures 3 and 4) had a stable basal fluorescence and generated Cts with an average of 34.89 and standard deviation of 0.46. Hence, improved optical properties imparted by inclusion of appropriate anti-foam can benefit any colorimetric or fluorescent analyte detection assay requiring optical measurement(s).

**EXAMPLE V: Optimal concentration of anti-foam for real-time quantitative PCR applications is dependent on composition.**

Real-time quantitative polymerase chain reactions specific for the human cytoplasmic β-actin sequence were carried out essentially as described above in Example 1 except that PCRs were formulated using separate component solutions as compared to a ready-to-use mastermix. Each 50-ul PCR contained 1.25 units of iTaq DNA polymerase (Bio-Rad Laboratories), 1X PCR buffer (20 mM Tris-HCl, pH 8.4, 50 mM KCl), 3 mM magnesium chloride, 0.2 mM each dNTP, 200 nM each primer, 100 nM FAM and TAMRA-labeled probe. PCRs were conducted under identical conditions except for the inclusion of varying amounts (0.1%, 0.01%, or 0.001%) of anti-foam selected from different commercially available preparations from Dow Coming (Antifoam AF, FG-10) or Sigma (SE-15) and different amounts of target DNA. Results are summarized in table 4.

Anti-foam concentration of 0.1% was inhibitory to PCR amplification with the selected set of anti-foam compounds resulting in either no amplification or delayed threshold cycle for product detection. Anti-foam concentrations of 0.01 % or lower did not inhibit PCR amplification. Additionally, examination of other compounds, namely DOW 1520-US, DOW Antifoam C, Sigma Antifoam B, or Sigma O-30, proved effective at all concentrations tested (0.1 %, 0.01%, 0.001%) (data not shown). These data illustrate how optimal anti-foam concentration may be determined empirically for any given compound, or mixture of agents, and or PCR application and reaction formulation.

**EXAMPLE VI Inclusion of anti-foam compound improves the precision and reliability of low-copy quantitative PCR.**

Real-time quantitative polymerase chain reactions specific for the human cytoplasmic β-actin sequence were carried out as essentially described above in example 3. A mastermix that was sufficient for 110, 50-μl reactions was prepared containing 1X iQ PCR SYBR Green SuperMix (Bio-Rad Laboratories), 300 nM each primer, and 20 copies of β-actin DNA template for each 50-μl reaction. This was divided into equal aliquots. Dow 1520-US anti-foam was added to a final concentration of 0.003% to one aliquot and an equivalent volume of water was added to the other. 50-μl aliquots from either mastermix were dispensed into each of 48 wells of a 96 well PCR plate using a multi-dispensing digital pipettor (Rainin). The plate was sealed as described in example 1 and cycled as described in example 3.

The average of Ct results for control reactions was 33.41 with a standard deviation of 1.29. The average of Ct results for reactions containing anti-foam was 32.62 with a standard deviation of 0.87. These data support the conclusion that addition of anti-foam improved precision and sensitivity of low copy PCR. Additionally, optical interference from bubbles in a limited number of control reactions required manual intervention in determining the best cycle range to use for baseline normalization of fluorescent signal (cycles 15 to 30) to achieve optimal Ct results for the control PCRs. Bubbles were absent in reactions that contained anti-foam.

Ideally, the basal fluorescence of a real-time reaction should be invariant from cycle to cycle until the accumulation of PCR product is sufficient to produce signal above background. Surfactants present in PCR and *Taq* DNA polymerase preparations, however, can result in bubbles when reactants are mixed. Failure to clear reactions of bubbles prior to PCR cycling can distort optical signals and skew background fluorescence readings. This effect is illustrated in the amplification plots for wells H2 and H5 presented in figures 1 and 2. A pronounced increase in fluorescence is visible near cycle 5 for well H2 and cycle 11 for well H5 (figure 1). The amplification plots presented in figure 2 demonstrate the effect of these fluorescent perturbations on Ct determination when they are included in the data set used to normalize all PCRs to a common baseline. Under these circumstances, the average Ct for these 6 reactions was 35.99 with a standard deviation of 2.19. In contrast, the 6 PCRs that contained anti-foam (figures 3 and 4) had a stable basal fluorescence and generated Cts with an average of 34.89 and standard deviation of 0.46. Hence, improved optical properties imparted by inclusion of appropriate anti-foam can benefit any colorimetric or fluorescent analyte detection assay requiring optical measurement(s).

**Example VII: Effect of antifoam agents on *in vitro* transcription:**

The effect of anti foam agents on enzymatic reactions for synthesis of RNA was studied by *in vitro* transcription using T7 RNA polymerase. *In vitro* transcription reactions were set up using commercially available reagent kits. Double stranded cDNA was prepared from rat brain RNA by standard cDNA methods (Superscript II, Invitrogen). The oligo dT primer used contained the T7 promoter sequence at its 5' end region, and therefore the double strand cDNA could be used as template for in vitro transcription using T7 RNA polymerase. The kit for T7 transcription was obtained from Quanta Biosciences, Inc., Rockville, MD, 20850. Transcription reactions were set up according to the manufacturer's instructions using 500 ng of rat brain cDNA as template. At different time points of incubation at 37 C 2 $\mu$L samples were removed from reaction, diluted into TE buffer and kept on ice. At the conclusion of incubation the amount of RNA synthesized was measured by Ribo Green fluorescent dye method(Molecular Probes, Eugene, OR).

The anti foam used in this example was a mixture of Sigma O-30 and Dow1520-US. The concentration tested were as follows:

**1X concentration:** 0.005% Sigma O-30 and 0.001% Dow 1520-US.
**2X concentration:** 0.01% Sigma O-30 and 0.002% Dow 1520-US.

As can be seen in Table 4, inclusion of antifoam in transcription did not interfere with T7 RNA polymerase activity and *in vitro* transcription of RNA. In addition presence of 2x antifoam resulted in faster kinetics of RNA synthesis and at the 2-hour time point there was a 10% increase in the amount of RNA synthesized.

<u>EMBODIMENTS</u>

Embodiment 1: A method for detecting a target nucleic acid in a sample, comprising the step of amplifying the target nucleic acid using a polymerase chain reaction, wherein said polymerase chain reaction is carried out in the presence of an effective amount of at least one anti-foam reagent that does not substantially inhibit the action of the polymerase.

Embodiment 2: The method according to embodiment 1, wherein said polymerase chain reaction is a quantitative polymerase chain reaction.

Embodiment 3: The method according to embodiment 2, wherein said polymerase chain reaction is a reverse transcriptase polymerase chain reaction.

Embodiment 4: The method according to any of embodiments 1-3, further comprising detecting the product of said polymerase chain reaction by optical detection.

Embodiment 5: The method according to embodiment 4, comprising detecting said product using a probe labeled with a detectable label.

Embodiment 6: The method according to embodiment 5, wherein said detectable label is a fluorescent dye.

Embodiment 7: The method according to embodiment 4, comprising detecting said product using a fluorescent nucleic acid-binding dye.

Embodiment 8: The method according to any of embodiments 1-7, wherein said polymerase chain reaction is carried out in the presence of an effective amount of at least two anti-foam reagents.

Embodiment 9: The method according to any of embodiments 1-7 wherein said anti-foam agent is selected from the group consisting of 1520-US, AF, FG-10, O-30, SE-15, and Antifoam B.

Embodiment 10: The method according to embodiment 8, wherein said at least two anti-foam reagents are selected from the group consisting of 1520-US, AF, FG-10, O-30, SE-15, and Antifoam B.

Embodiment 11: A composition for amplifying a target nucleic acid, comprising

(a) at least one primer molecule that hybridizes to the target nucleic acid;
(b) nucleotide triphosphates
(c) a thermostable DNA polymerase
(d) a detergent; and
(e) an effective amount of at least one anti-foam reagent that does not substantially inhibit the action of said thermostable DNA polymerase.

Embodiment 12: A composition according to embodiment 11, comprising at least two anti- foam reagents.

Embodiment 13: A composition according to embodiment 11 wherein said anti-foam agent is selected from the group consisting of 1520-US, AF, FG-10, O-30, SE-15, and Antifoam B.

Embodiment 14: The composition according to embodiment 12, wherein said at least two anti-foam reagents are selected from the group consisting of 1520-US, AF, FG-10, O-30, SE-15, and Antifoam B.

Embodiment 15: The method according to embodiment 1 wherein said polymerase chain reaction is carried out in a sample chamber of a device comprising a plurality of said sample chambers.

Embodiment 16: The method according to embodiment 15, wherein each of a plurality of said sample chambers of said device contains reagents suitable for detecting a target nucleic acid.

Embodiment 17: The method according to embodiment 16, wherein a plurality of sample chambers of said device contains reagents suitable for detecting different target nucleic acids.

Embodiment 18: The method according to embodiment 17, further comprising detecting the amplified products in said sample chambers by optical detection.

Embodiment 19: The method according to embodiment 18, comprising detecting said amplified products using a probe labeled with a detectable label.

Embodiment 20: The method according to embodiment 19, wherein said detectable label is a fluorescent dye.

Embodiment 21: The method according to embodiment 18, comprising detecting said amplified products using a fluorescent nucleic acid-binding dye.

**Claims**

1. A method for detecting a target nucleic acid in a sample, comprising the step of amplifying the target nucleic acid using a polymerase chain reaction, wherein said polymerase chain reaction is carried out in the presence of an effective amount of at least one anti-foam reagent that does not substantially inhibit the action of the polymerase.

2. The method according to claim 1, wherein said polymerase chain reaction is a quantitative polymerase chain reaction.

3. The method according to claim 2, wherein said polymerase chain reaction is a reverse transcriptase polymerase chain reaction.

4. The method according to any of claims 1-3, further comprising detecting the product of said polymerase chain reaction by optical detection.

5. The method according to claim 4, comprising detecting said product using a probe labeled with a detectable label.

6. The method according to any of claims 1-5, wherein said polymerase chain reaction is carried out in the presence of an effective amount of at least two anti-foam reagents.

7. The method according to any of claims 1-5 wherein said anti-foam agent is selected from the group consisting of 1520-US, AF, FG-10, O-30, SE-15, and Antifoam B.

8. A composition for amplifying a target nucleic acid, comprising

    (a) at least one primer molecule that hybridizes to the target nucleic acid;
(b) nucleotide triphosphates
(c) a thermostable DNA polymerase
(d) a detergent; and
(e) an effective amount of at least one anti-foam reagent that does not substantially inhibit the action of said thermostable DNA polymerase.

9. A composition according to claim 8 wherein said anti-foam agent is selected from the group consisting of 1520-US, AF, FG-10, O-30, SE-15, and Antifoam B.

10. The method according to claim 1 wherein said polymerase chain reaction is carried out in a sample chamber of a device comprising a plurality of said sample chambers.

11. The method according to claim 10, wherein each of a plurality of said sample chambers of said device contains reagents suitable for detecting a target nucleic acid.

12. The method according to claim 11, wherein a plurality of sample chambers of said device contains reagents suitable for detecting different target nucleic acids.

13. The method according to claim 12, further comprising detecting the amplified products in said sample chambers by optical detection.

14. The method according to claim 13, comprising detecting said amplified products using a probe labeled with a detectable label.

15. The method according to claim 13, comprising detecting said amplified products using a fluorescent nucleic acid-binding dye.

| DNA Target Amount (copies) | Threshold Cycle (Ct) Results Concentration of DOW 1520-US | | | | |
|---|---|---|---|---|---|
| | 0% | 0.1% | 0.01% | 0.001% | 0.0001% |
| $1 \times 10^2$ | 35.19 35.43 35.27 | 33.667 33.97 34.195 | 35.403 33.979 33.743 | 34.723 33.363 34.012 | 35.604 33.907 33.409 |
| $1 \times 10^4$ | 27.8 27.93 28.02 | 26.557 27.079 27.208 | 26.937 26.867 27.275 | 27.051 27.131 27.183 | 27.11 27.202 27.489 |
| $1 \times 10^6$ | 21.04 20.94 21.25 | 20.207 19.916 20.419 | 20.495 20.302 20.707 | 20.501 20.613 20.613 | 20.753 20.575 20.661 |
| $1 \times 10^8$ | 13.87 13.96 13.8 | 13.736 13.642 13.708 | 13.716 13.816 13.935 | 13.76 13.804 13.934 | 14.003 13.931 14.018 |
| | | | | | |
| Standard Curve Slope | -3.555 | -3.376 | -3.409 | -3.357 | -3.379 |
| Correlation Coefficient | -1.000 | -0.9995 | -0.9984 | -0.9992 | -0.9979 |
| PCR efficiency | 91.1% | 97.8% | 96.5% | 98.5% | 97.7% |

**Table 1.** Effect of Dow 1520-US anti-foam on Taqman real-time quantitative PCR. Summary of Ct values for Taqman PCRs containing varying amounts of anti-foam and DNA target as indicated in the table and corresponding linear regression analysis for the Ct versus log DNA input standard curve.

| Amount of Target DNA (copies) | Threshold Cycle (Ct) Results | | | | | | |
|---|---|---|---|---|---|---|---|
| | CNTRL | DOW 1520-US | DOW AF | DOW FG-10 | Sigma O-30 | Sigma SE-15 | Sigma B |
| $1\times10^2$ | 35.248 | 34.39 | 34.244 | 33.79 | 35.744 | 35.234 | 33.773 |
| | 35.512 | 33.896 | 33.895 | 33.049 | 34.588 | 34.369 | 34.115 |
| | 35.347 | 34.286 | 32.268 | 34.714 | 34.716 | 34.544 | 35.803 |
| $1\times10^4$ | 27.891 | 27.177 | 26.469 | 26.895 | 27.675 | 27.292 | 27.928 |
| | 28.012 | 27.488 | 26.743 | 26.584 | 27.314 | 26.994 | 27.467 |
| | 28.113 | 27.415 | 26.551 | 26.706 | 27.274 | 27.131 | 27.125 |
| $1\times10^6$ | 21.127 | 20.675 | 19.975 | 20.115 | 20.997 | 20.249 | 20.44 |
| | 21.044 | 20.354 | 20.003 | 20.133 | 20.804 | 20.152 | 20.653 |
| | 21.339 | 20.654 | 20.03 | 20.242 | 20.959 | 20.298 | 20.969 |
| $1\times10^8$ | 13.952 | 13.664 | 13.344 | 13.694 | 13.685 | 13.602 | 13.91 |
| | 14.046 | 13.848 | 13.66 | 13.694 | 13.912 | 13.476 | 13.738 |
| | 13.872 | 13.958 | 13.319 | 13.699 | 13.878 | 13.696 | 14.01 |
| | | | | | | | |
| Standard Curve Slope | -3.554 | -3.393 | -3.333 | -3.352 | -3.504 | -3.514 | -3.443 |
| Correlation. Coefficient | -1.000 | -1.000 | 0.998 | -0.999 | -0.999 | -0.999 | -0.998 |
| PCR Efficiency | 91.2% | 97.1% | 99.5% | 98.8% | 92.2% | 92.6% | 95.2% |

**Table 2.** Effect of anti-foam compounds on TaqMan real-time quantitative PCR. Summary of Ct values for Taqman PCRs containing various anti-foam compounds, or control reactions that omit anti-foam (CNTRL), and DNA target as indicated in the table and corresponding linear regression analysis for the Ct versus log DNA input standard curve.

| Amount of Target DNA (copies) | Threshold Cycle (Ct) Results | | | | | | |
|---|---|---|---|---|---|---|---|
| | CNTRL | DOW 1520-US | DOW AF | DOW FG-10 | Sigma O-30 | Sigma SE-15 | Sigma B |
| $1 \times 10^2$ | 34.111 | 33.8220 | 34.665 | 34.283 | 33.793 | 33.805 | 34.708 |
| | 33.876 | 33.1470 | 33.946 | 33.769 | 33.565 | 34.218 | 33.748 |
| | 34.826 | 32.9940 | 34.584 | 34.066 | 32.444 | 34.662 | 33.106 |
| $1 \times 10^4$ | 26.114 | 25.5990 | 26.384 | 25.848 | 25.432 | 25.903 | 25.866 |
| | 26.339 | 25.9960 | 25.909 | 25.483 | 25.121 | 26.286 | 25.535 |
| | 26.806 | 26.0230 | 26.118 | 25.917 | 25.172 | 26.215 | 25.648 |
| $1 \times 10^6$ | 18.68 | 18.2440 | 18.501 | 18.559 | 17.876 | 18.336 | 18.253 |
| | 18.059 | 18.1490 | 18.534 | 18.186 | 17.959 | 18.371 | 18.175 |
| | 18.528 | 18.2630 | 18.593 | 18.359 | 18.048 | 18.939 | 18.607 |
| $1 \times 10^8$ | 11.498 | 11.5170 | 11.775 | 11.601 | 11.455 | 11.980 | 11.835 |
| | 11.755 | 11.7380 | 11.835 | 11.565 | 11.398 | 11.823 | 11.763 |
| | 11.923 | 11.8190 | 11.905 | 11.846 | 11.534 | 11.888 | 11.678 |
| | | | | | | | |
| Standard Curve Slope | -3.782 | -3.6270 | -3.764 | -3.724 | -3.635 | -3.729 | -3.681 |
| Correlation. Coefficient | -0.999 | -0.9990 | -0.999 | -0.999 | -0.998 | -0.999 | -0.998 |
| PCR Efficiency | 83.8% | 88.7% | 84.4% | 85.6% | 88.4% | 85.4% | 86.9% |

**Table 3.** Effect of anti-foam compounds on SYBR Green I real-time quantitative PCR. Summary of Ct values for real-time PCRs containing various anti-foam compounds, or control reactions that omit anti-foam (CNTRL), and DNA target as indicated in the table and corresponding linear regression analysis for the Ct versus log DNA input standard curve.

| Rx time | Control without antifoam | Control without antifoam | 1x antifoam | 1x antifoam | 2x antifoam |
|---|---|---|---|---|---|
| 2 Hour | 22620 | 21124 | 23299 | 23646 | 24754 |
| | 21867 | 20709 | 22765 | 22848 | 24340 |
| | | | | | |
| 4 Hour | 46872 | 47293 | 47619 | 46919 | 46791 |
| | 46685 | 45771 | 45231 | 44173 | 44722 |

**Table 4:** Effect of antifoam agents on in vitro transcription: Invitro transcription reactions were set up using commercially available reagent kits. Double stranded cDNA was prepared from rat brain RNA by standard cDNA methods (Superscript II, Invitrogen ). The oligo dT primer used contained the T7 promoter sequence at its 5' end region, therefore the double strand cDNA could be used as template for in vitro transcription using T7 RNA polymerase. The kit for T7 transcription was obtained from Quanta Biosciences, Inc., Rockville, MD, 20850. Transcription reactions were set up according to the manufacturer's instructions using 500 ng of rat brain cDNA as template. At different time points of incubation at 37 C 2 uL samples were removed from reaction, diluted into TE buffer and were kept on ice. At the conclusion of incubation the amount of RNA synthesized was measured by Ribo Green fluorescent dye method(Molecular Probes, Eugene, OR) Fluorescence measurements for each data point and reaction is shown in the table.

**Figure 1.** Effect of surfactant foaming on fluorescent signal of real-time PCR of low copy template. Plot of raw relative fluorescence readings collected at each cycle during PCR of 20 copies β-actin template, amplified in the presence of SYBR Green I, for 6 representative reactions from a 48-reaction set. Perturbation to basal fluorescence is evident in plots for PCRs from well H2 and H5.

**Figure 2.** Effect of surfactant foaming on threshold cycle (Ct) determination in real-time PCR of low copy template. Plot of baseline normalized relative fluorescence readings collected at each cycle during PCR of 20 copies β-actin template, amplified in the presence of SYBR Green I, for 6 representative reactions from a 48 reaction set. Perturbation to basal fluorescence in PCRs for wells H2 and H5 results in distortion of baseline and aberrant determination of threshold cycle (Ct) with poor precision. Range of Ct values indicated by grey box.

**Figure 3.** Control of surfactant foaming by anti-foam enables stable basal fluorescence during real-time PCR of low copy template. Plot of raw relative fluorescence readings collected at each cycle during PCR of 20 copies β-actin template, amplified in the presence of SYBR Green I and 0.003% Dow 1520-US anti-foam, for 6 representative reactions (wells H7 – H12) from a 48 reaction set.

**Figure 4.** Anti-foam improves precision of Ct results for real-time PCR of low copy template. Plot of baseline normalized relative fluorescence readings collected at each cycle during PCR of 20 copies β-actin template, amplified in the presence of SYBR Green I and 0.003% Dow 1520-US anti-foam, for 6 representative reactions (wells H7 – H12) from a 48 reaction set. Range of Ct values indicated by grey box.

Europäisches
Patentamt

European
Patent Office

Office européen
des brevets

# EUROPEAN SEARCH REPORT

Application Number

EP 10 01 2272

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 5 928 905 A (STEMMER WILLEM P C [US] ET AL) 27 July 1999 (1999-07-27)<br>* column 3, lines 30-43 *<br>* column 12, lines 11-58 *<br>* column 19, lines 48-56 *<br>----- | 1,4,6<br><br>2,3,5,<br>7-15 | INV.<br>C12Q1/68 |
| X,D<br>Y | US 5 985 569 A (FOXALL PAUL A [US] ET AL) 16 November 1999 (1999-11-16)<br>* abstract *<br>* examples 7,10 *<br>----- | 8<br><br>1-7,9-15 | |
| X,D<br>Y | US 5 962 273 A (DURMOWICZ GERARD P [US] ET AL) 5 October 1999 (1999-10-05)<br>* the whole document *<br>* example 14 *<br>----- | 8<br><br>1-7,9-15 | |
| X<br>Y | US 5 763 185 A (COLLIS MATTHEW P [US] ET AL) 9 June 1998 (1998-06-09)<br>* the whole document *<br>* example 1 *<br>----- | 8<br><br>1-7,9-15 | |
| Y | HEID C A ET AL: "REAL TIME QUANTITATIVE PCR",<br>GENOME RESEARCH, COLD SPRING HARBOR LABORATORY PRESS, WOODBURY, NY, US,<br>vol. 6, no. 10, October 1996 (1996-10), pages 986-994, XP000642795,<br>ISSN: 1088-9051<br>* the whole document *<br>----- | 1-15 | TECHNICAL FIELDS SEARCHED (IPC)<br><br>C12Q |

The present search report has been drawn up for all claims

| Place of search | Date of completion of the search | Examiner |
|---|---|---|
| Munich | 7 June 2011 | Sauer, Tincuta |

EPO FORM 1503 03.82 (P04C01)

EP 2 345 738 A1

## ANNEX TO THE EUROPEAN SEARCH REPORT
## ON EUROPEAN PATENT APPLICATION NO.

EP 10 01 2272

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2011

| Patent document cited in search report | | Publication date | Patent family member(s) | | Publication date |
|---|---|---|---|---|---|
| US 5928905 | A | 27-07-1999 | NONE | | |
| US 5985569 | A | 16-11-1999 | NONE | | |
| US 5962273 | A | 05-10-1999 | AU 740879 B2 | | 15-11-2001 |
| | | | AU 9048198 A | | 27-05-1999 |
| | | | CA 2249635 A1 | | 04-05-1999 |
| | | | DE 69807030 D1 | | 12-09-2002 |
| | | | DE 69807030 T2 | | 12-12-2002 |
| | | | EP 0919633 A2 | | 02-06-1999 |
| | | | ES 2178092 T3 | | 16-12-2002 |
| | | | JP 4209977 B2 | | 14-01-2009 |
| | | | JP 11225781 A | | 24-08-1999 |
| US 5763185 | A | 09-06-1998 | AU 728014 B2 | | 04-01-2001 |
| | | | AU 4931497 A | | 02-07-1998 |
| | | | BR 9706374 A | | 01-06-1999 |
| | | | CA 2220270 A1 | | 30-06-1998 |
| | | | DE 69722159 D1 | | 26-06-2003 |
| | | | DE 69722159 T2 | | 27-11-2003 |
| | | | EP 0851032 A1 | | 01-07-1998 |
| | | | ES 2197294 T3 | | 01-01-2004 |
| | | | JP 4271273 B2 | | 03-06-2009 |
| | | | JP 10191978 A | | 28-07-1998 |
| | | | US 6210881 B1 | | 03-04-2001 |

EPO FORM P0459

For more details about this annex : see Official Journal of the European Patent Office, No. 12/82

## REFERENCES CITED IN THE DESCRIPTION

### Patent documents cited in the description

- US 40068502 P
- EP 50424 A, Erlich H.
- EP 84796 A
- EP 258017 A
- EP 237362 A
- EP 201184 A, Mullis, K.
- US 4683202 A, Mullis K.
- US 4582788 A, Erlich, H.
- US 4683194 A, Saiki, R.
- US 6127155 A, Gelfand
- US 6027923 A, Wallace
- US 2000 A
- US 5985569 A
- US 5962273 A
- WO 9206200 A
- WO 9610640 A
- US 5436149 A
- US 4962020 A
- US 5173411 A
- US 5498523 A

### Non-patent literature cited in the description

- **MULLIS, K. et al.** *Cold Spring Harbor Symp. Quant. Biol.,* 1986, vol. 51, 263-273
- **SIEBERT.** Molecular Diagnosis of infectious diseases. Humana Press, 1998, 55-79
- **WIESNER et al.** *Nucl. Acids Res.,* 1992, vol. 20, 5863-5864
- **HOLLAND et al.** *Proc. Natl. Acad. Sci. U.S.A.,* 1991, vol. 88, 7276
- **GIBSON et al.** *Genome Res.,* 1996, vol. 6, 99
- **HEID et al.** *Genome Res.,* 1996, vol. 6, 986
- **TYAGI, S. ; KRAMER, F.R.** Molecular Beacons. *Nature Biotechnology,* 1996, vol. 14, 303
- **NAZARENKO et al.** *Nucleic. Acids Res.,* 1997, vol. 25, 2516
- **NAZERENKO et al.** *Nucleic. Acids Res.,* 2002
- **HIGUCHI et al.** *Biotechnology,* 1992
- **HIGUCHI et al.** *Biotechnology,* 1993, vol. 11 (102610), 413
- **ISHIGURO et al.** *Anal. Biochem.,* 1995, vol. 229, 207
- **WITTWER et al.** *Biotechniques,* 1997, vol. 22, 130
- **HIGUCHI R ; G DOLLINGER ; P S WALSH ; R. GRIFFITH.** Simultaneous amplification and detection of specific DNA sequences. *Bio/Technology,* 1992, vol. 10, 413-417
- **HIGUCHI R ; C FOCKLER ; G DOLLINGER ; R WATSON.** Kinetic PCR analysis: real time monitoring of DNA amplification reactions. *Bio/Technology,* vol. 11, 1026-1030
- **KARSAI et al.** *BioTechniques,* 2002, vol. 32, 790
- *LCR, PROCEEDINGS OF THE NATIONAL ACADEMY OF SCIENCE, USA,* 1991, vol. 88, 189
- *NASBA, NATURE,* 1991, vol. 350, 91
- *3SR, PROCEEDINGS OF THE NATIONAL ACADEMY OF SCIENCE, USA,* 1990, vol. 87, 1874
- **HARLOW, E. ; LANE, D.** Antibodies: A Laboratory Manual. Cold Spring Harbor Laboratory Press, 1988
- **KAUFINAN, P. B. et al.** Handbook of Molecular and Cellular Methods in Biology and Medicine. CRC Press, 1995, 468-469
- **KOHLER et al.** *Nature,* 1975, vol. 256, 495
- **KOHLER et al.** *Eur. J Immunol.,* 1976, vol. 6, 511
- **KOHLER et al.** *Eur. J. Immunol.,* 1976, vol. 6, 292
- **HAMMERLING et al.** Monoclonal Antibodies and T-Cell Hybridomas. Elsevier, 1981, 563-681
- **KAUFINAN, P. B. et al.** Handbook of Molecular and Cellular Methods in Biology and Medicine. CRC Press, 1995, 444-467
- **XU et al.** *Focus,* 2000, vol. 22, 3-5